# EUROPEAN PATENT SPECIFICATION

(11) **EP 2 243 427 B1**
(45) Date of publication and mention of the grant of the patent: **24.04.2013**
(21) Application number: 09713586.7
(22) Date of filing: 20.02.2009
(51) Int. Cl.: A61B 5/151

(54) **PUNCTURE TOOL WITH MECHANISM FOR RELIEVING NEEDLE PUNCTURE PAIN AND TOOL FOR RELIEVING NEEDLE PUNCTURE PAIN**
PUNKTIERUNGSWERKZEUG MIT MECHANISMUS ZUR LINDERUNG VON NADELPUNKTURSCHMERZEN SOWIE WERKZEUG ZUR LINDERUNG VON NADELPUNKTURSCHMERZEN
OUTIL DE PONCTION AVEC MÉCANISME POUR SOULAGER UNE DOULEUR DE PONCTION D'AIGUILLE ET OUTIL POUR SOULAGER UNE DOULEUR DE PONCTION D'AIGUILLE

(30) Priority: 21.02.2008 JP 2008040605; 12.03.2008 JP 2008063267
(43) Date of publication of application: 27.10.2010
(73) Proprietor: Terumo Kabushiki Kaisha, Tokyo 151-0072 (JP)
(72) Inventor: KUBOTERA, Yukinori, Ashigarakamigun Kanagawa 259-0151 (JP)
(74) Representative: TBK
(86) International application number: PCT/JP2009/053089
(87) International publication number: WO 2009/104765

(56) References cited:
- EP-A1- 1 493 386
- EP-A1- 1 535 572
- EP-A1- 1 591 065
- WO-A1-2004/021886
- JP-A- 2001 314 395
- JP-A- 2006 075 369
- JP-A- 2007 330 509
- US-A1- 2004 073 140

## Description

### Technical Field

The present invention relates to a puncture tool with mechanism for relieving needle puncture pain and a tool for relieving needle puncture pain.

### Background of the Invention

In recent years, along with increased diabetic patients, blood glucose self-measurement has been recommended in which a patient monitors daily variations in blood glucose level by oneself.

The blood glucose level is measured using a test paper giving a color in response to a quantity of glucose in blood or blood glucose measuring equipment using a biosensor or the like in which an electric current increases according to the quantity of glucose in blood.

A method in which a patient collects own blood before the measurement involves puncturing a skin (surface) of a fingertip (finger) pulp by use of a puncture tool equipped with a puncture needle or a cutter and thereafter pressing the periphery of the puncture portion with fingers to squeeze blood.

Such a puncture tool is configured to include a puncture tool body and a plunger housed in the puncture tool body and to attach a puncture needle to the plunger. The plunger is designed to be displaced from a waiting position where puncture is prepared to a puncture position where the puncture is done. A delivery (operating) button is pressed so that the plunger is displaced from the waiting position to the puncture position. Thus, the puncture needle punctures the skin of the fingertip.

In order to control the blood glucose level, it is necessary to measure the blood glucose level a plurality of times in a day. In each case the puncture is done with the puncture tool. The puncture sites are changed every time so that the skin surface condition can be maintained satisfactorily.

Incidentally, a patient (user) feels a pain when the surface of a fingertip pulp is punctured with a puncture tool. Therefore, it is desired to relieve the pain. To meet the desire, a puncture tool as below is proposed. The puncture tool is provided with a vibrating member. The puncture is done while the vibrating member is brought into contact with the vicinity of a puncture-planned site on the surface of the fingertip pulp to apply vibrations thereto, thereby relieving a pain. (See patent document 1.)

However, such a puncture tool has the following defect. When the puncture is done, a wound (puncture wound) formed on the surface of the fingertip pulp by the puncture (e.g. the previous puncture) done previously or in the site immediately close to such a wound has to be avoided. Therefore, the vibrating member actually comes into contact with the puncture wound or the periphery thereof to apply vibrations actually thereto, which increases the pain of the wound.

Patent Document 1: Japanese Patent Laid-open No. 2005-46612

Document EP 1535572 A1 discloses an invasive apparatus comprising a contact portion having a vibrating body. One embodiment shows a pair of clip pieces being able to sandwich a part of the finger's tissue.

Document US 2004/0073140 A1 discloses a stimulator member that is adapted to engage a skin surface and to stretch open an incision of the skin surface.

Document EP 1591065 A1 discloses a vibration sensor which analyses a received output signal and sends an actuation signal to a bodily fluid extraction device.

### Disclosure of Invention

It is an object of the present invention to provide a puncture tool with mechanism for relieving puncture pain and a tool for relieving needle puncture pain that can relieve a pain occurring when the surface of a finger pulp is punctured with a puncture needle.

To achieve the above object, the present invention provides a needle puncture pain relieving tool according to claim 1 and a puncture tool (device) according to claim 7. Preferable embodiments are set forth in the dependent claims. Preferably, a puncture device (tool) with mechanism for relieving needle puncture pain has a puncture tool used to puncture a surface of a finger pulp with a puncture needle and needle puncture pain relieving means for relieving a pain occurring when the surface of the finger pulp is punctured with the puncture needle, **characterized in that** the needle puncture pain relieving means includes: a puncture portion faicing to the finger pulp; a contact portion oppositely disposed via the puncture portion and coming into contact with a lateral portion of the finger; and a vibrator having a vibrating body applying vibratory stimulations to a lateral portion of the finger and driving the vibrating body.

With this, because of having the needle puncture pain relieving means, a pain occurring when the surface of the finger pulp is punctured with the puncture needle can be relieved.

Since the pain is relieved by applying the vibratory stimulations to the lateral portions of the finger, it is possible to prevent the increase of the pain of the wound (the puncture wound) formed on the front surface of the finger pulp by the previous puncture.

In the puncture tool with mechanism for relieving needle puncture pain, preferably, the contact portion includes a clip portion having a pair of clip pieces, the clip pieces being disposed to face each other via the puncture portion and coming into contact with the respective opposite portions of the finger; and the vibrating body is provided on at least one of the clips.

With this, the vibratory stimulations can more positively be applied to the lateral portions of the finger.

In the puncture tool with mechanism for relieving needle puncture pain, preferably, the vibrating body is disposed on a surface side, of the clip piece, coming into contact with a lateral portion of the finger.

With this, the vibratory stimulations can efficiently be applied to the lateral portion of the finger.

In the puncture tool with mechanism for relieving needle puncture pain, preferably, the vibrating body is provided on each of the clip pieces.

With this, since the vibratory stimulations can be applied to the opposite portions of the finger, the pain can more positively be relieved.

In the puncture tool with mechanism for relieving needle puncture pain, preferably, a portion, of the clip piece, coming into contact with the finger has on a front surface thereof a curved concave surface corresponding to a shape of the finger.

With this, the clip pieces can accurately come into contact with and grip the lateral portions of the fingertip.

In the puncture tool with mechanism for relieving needle puncture pain, preferably, a portion, of the clip piece, coming into contact with the lateral portion of the finger is formed with concavities and convexities on a front surface.

With this, the clip pieces come into point-contact with the respective lateral portions of the fingertip, thereby further relieving the pain.

In the puncture tool with mechanism for relieving needle puncture pain, preferably, the needle puncture pain relieving means includes a base portion secured to the puncture tool and a pair of elastically deformable portions each connecting the base portion with a corresponding one of the clip pieces.

With this, the elastic force of the pair of the elastically deformable portions allows the pair of clip pieces to grip and press the fingertip from their opposite sides. In this way, the periphery of the puncture portion of the fingertip is squeezed to allow the wound to bleed moderately. Thus, it is possible to collect a necessary quantity of blood easily and rapidly. Additionally, it becomes easy to transmit vibrations from the piezoelectric elements to the corresponding lateral portions of the fingertip. Thus, vibratory stimulations can be applied to the opposite portions of the fingertip efficiently.

In the puncture tool with mechanism for relieving needle puncture pain, preferably, the puncture portion has a contact portion coming into contact with the finger and a passage through which the puncture needle is passed.

With this, when the surface of the finger pulp is punctured with the puncture needle, the puncture can be done reliably.

In the puncture tool with mechanism for relieving needle puncture pain, preferably, the contact portion has on a front surface thereof a curved concave surface corresponding to a shape of the finger.

With this, the contact portion can be brought into contact with the fingertip accurately.

In the puncture tool with mechanism for relieving needle puncture pain, preferably, the needle puncture pain relieving means has a control section adapted to control the operation of the vibrator.

With this, for example, control can variously be exercised to automatically actuate or stop the vibrator and to vary the level of the vibration of the vibrating body with time.

In the puncture tool with mechanism for relieving needle puncture pain, preferably, the needle puncture pain relieving means has detection means for detecting a predetermined condition of the puncture tool with mechanism for relieving puncture pain and the control section is configured to actuate and/or stop the vibrator on the basis of the detection result of the detection means.

With this, since the vibrations of the piezoelectric elements are automatically started and stopped, the operation is facilitated, providing improved convenience.

In the puncture tool with mechanism for relieving needle puncture pain, preferably, the control section is configured to vary the level of the vibrations of the vibrating body with time.

With this, since a user's sensation concentrates on the variation of the vibratory level of the vibrating body, the user lowers ability to perceive the pain, thereby further relieving the pain.

Preferably, the puncture tool with mechanism for relieving needle puncture pain has informing means for prompting the user of the puncture tool to count the number of variations of the vibratory level of the vibrating body.

With this, while the user counts the number of the variations of the vibratory level of the vibrating body, the user's sensation concentrates on it, so that the user lowers the ability to perceive a pain, further relieving the pain.

In the puncture tool with mechanism for relieving needle puncture pain, preferably, the vibratory frequency of the vibrating body is 1000 Hz or lower.

With this, the vibratory stimulations with necessary and sufficient magnitude can be applied to the user's fingertip safely and reliably.

In the puncture tool with mechanism for relieving needle puncture pain, preferably, the vibratory amplitude of the vibrating body is 6 µm or greater.

With this, the vibratory stimulations with necessary and sufficient magnitude can be applied to the user's fingertip safely and reliably.

In the puncture tool with mechanism for relieving needle puncture pain, preferably, the puncture tool includes a puncture tool body; a plunger to which the puncture needle is detachably attached and which is displaceable between a waiting position where the puncture needle is prepared to puncture the surface of the finger pulp and a puncture position where the puncture is done; and a puncture operating portion operated to start the displacement of the plunger from the waiting position to puncture position.

With this, the surface of the finger pulp can be punctured easily and reliably.

Preferably, a needle puncture pain relieving tool is used along with a puncture tool to puncture a surface of a finger pulp with a puncture needle to relieve a pain occurring when the surface of the finger pulp is punctured with the puncture needle, including: a contact portion coming into contact with the opposite sides of the finger; and a vibrator having a vibrating body applying vibratory stimulations to the side portion of the finger and driving the vibrating body.

With this, a pain occurring when the surface of the finger pulp is punctured with the puncture needle can be relieved. In addition, since the pain is relieved by applying the vibratory stimulations to the lateral portions of the finger, it is possible to prevent the increase of the pain of the wound (the puncture wound) formed on the front surface of the finger pulp by the previous puncture.

In the needle puncture pain relieving tool, preferably, the contact portion includes a clip portion having a pair of clip pieces disposed to face each other and coming into contact with the respective opposite sides of the finger and the vibrating body is provided on at least one of the clips.

With this, the vibratory stimulations can be applied to the lateral portions of the finger more reliably.

In the needle puncture pain relieving tool, preferably, the vibrating body is disposed on a surface side, of the clip piece, coming into contact with a lateral portion of the finger.

With this, the vibratory stimulations can be applied to a lateral portion of the finger efficiently.

In the needle puncture pain relieving tool, preferably, the vibrating body is provided on each of the clip pieces.

With this, the vibratory stimulations can be applied to the opposite sides of the finger; therefore, the wound can be reduced more reliably.

In the needle puncture pain relieving tool, preferably, a portion, of the clip piece, coming into contact with the finger has on a front surface thereof a curved concave surface corresponding to a shape of the finger.

With this, clip pieces can accurately come into contact with the lateral portions of the fingertip to grip the fingertip.

In the needle puncture pain relieving tool, preferably, a portion, of the clip pieces, coming into contact with the lateral portions of the finger is formed with concavities and convexities on the surface.

With this, the clip pieces come into pint-contact with the lateral portions of the fingertip, which further relieves the pain.

Preferably, the needle puncture pain relieving tool includes a puncture portion opposed to the finger pulp and the pair of clip pieces are disposed to face each other via the puncture portion.

With this, the vibratory stimulations can be applied to the lateral portions of the finger.

In the needle puncture pain relieving tool, preferably, the puncture portion has a contact portion coming into contact with the finger and a passage through which the puncture needle is passed.

With this, when the surface of the finger pulp is punctured with the puncture needle, the puncture can be done reliably.

In the needle puncture pain relieving tool, preferably, the contact portion has on a front surface thereof a curved concave surface corresponding to a shape of the finger.

With this, the contact portion can be brought into contact with the fingertip accurately.

Preferably, the needle puncture pain relieving tool includes a base portion secured to the puncture tool and a pair of elastically deformable portions each connecting the base portion with a corresponding one of the clip pieces.

With this, the elastic force of the pair of the elastically deformable portions allows the pair of clip pieces to grip and press the fingertip from their opposite sides. In this way, the periphery of the puncture portion of the fingertip is squeezed to allow the wound to bleed moderately. Thus, it is possible to collect a necessary quantity of blood easily and rapidly. Additionally, it becomes easy to transmit vibrations from the piezoelectric elements to the corresponding lateral portions of the fingertip. Thus, vibratory stimulations can be applied to the lateral portions of the fingertip efficiently.

In the needle puncture pain relieving tool, preferably, the needle puncture pain relieving tool is configured to be detachably attached to the puncture tool.

With this, the puncture tool and the needle puncture pain relieving tool can be used in the integral state, which facilitates the operation.

Preferably, the needle puncture pain relieving tool has a control section adapted to control the operation of the vibrator.

With this, for example, control can variously be exercised to automatically actuate or stop the vibrator and to vary the vibratory level of the vibrating body with time.

In the needle puncture pain relieving tool, preferably, the control section is configured to vary the vibratory level of the vibrating body with time.

With this, since a user's sensation concentrates on the variation of the vibratory level of the vibrating body, the user lowers ability to perceive the pain, thereby further relieving the pain.

Preferably, the needle puncture pain relieving tool has informing means for prompting the user of the puncture tool to count the number of variations of the vibratory level of the vibrating body.

With this, while the user counts the number of the variations of the vibratory level of the vibrating body, the user's sensation concentrates on it, so that the user lowers the ability to perceive a pain, further reducing the pain.

In the needle puncture pain relieving tool, preferably, the vibratory frequency of the vibrating body is 1000 Hz or lower.

With this, the vibratory stimulations with necessary and sufficient magnitude can be applied to the user's fingertip safely and reliably.

In the needle puncture pain relieving tool, preferably, the vibratory amplitude of the vibrating body is 6 µm or greater.

With this, the vibratory stimulations with necessary and sufficient magnitude can be applied to the user's fingertip safely and reliably.

In the needle puncture pain relieving tool, preferably, the puncture tool includes a puncture tool body; a plunger to which the puncture needle is detachably attached and which is displaceable between a waiting position where the puncture needle is prepared to puncture the surface of the finger pulp and a puncture position where the puncture is done; and a puncture operating portion operated to start the displacement of the plunger from the waiting position to puncture position.

With this, the surface of the finger pulp can be punctured easily and reliably.

### Brief Description of Drawings

[FIG. 1]

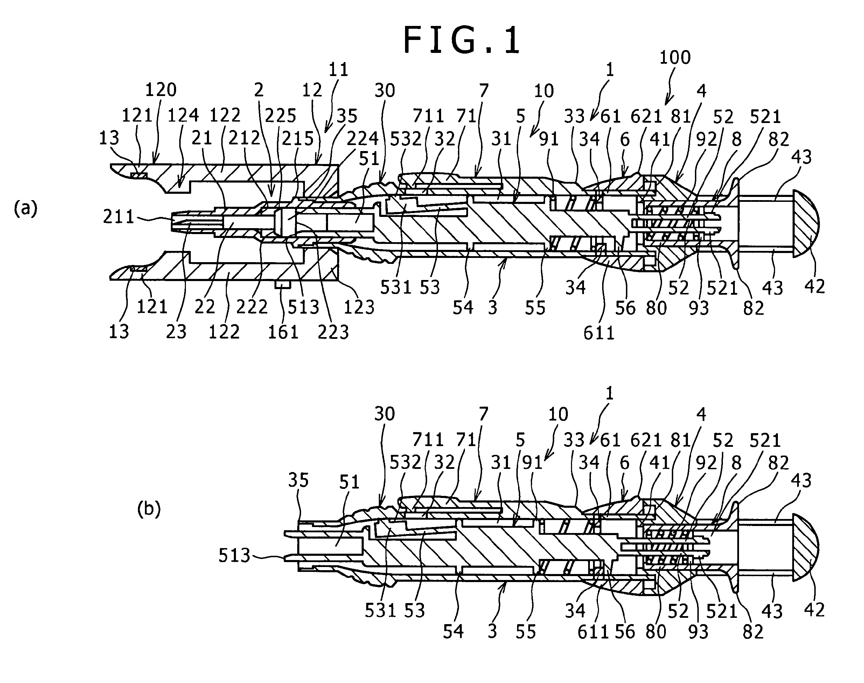

   FIG. 1 is a perspective view illustrating a first embodiment of a puncture tool with mechanism for relieving needle puncture pain of the present invention (the first embodiment of a needle puncture pain relieving tool and a puncture tool attached with the needle puncture pain relieving tool in the present invention).
[FIG. 2]

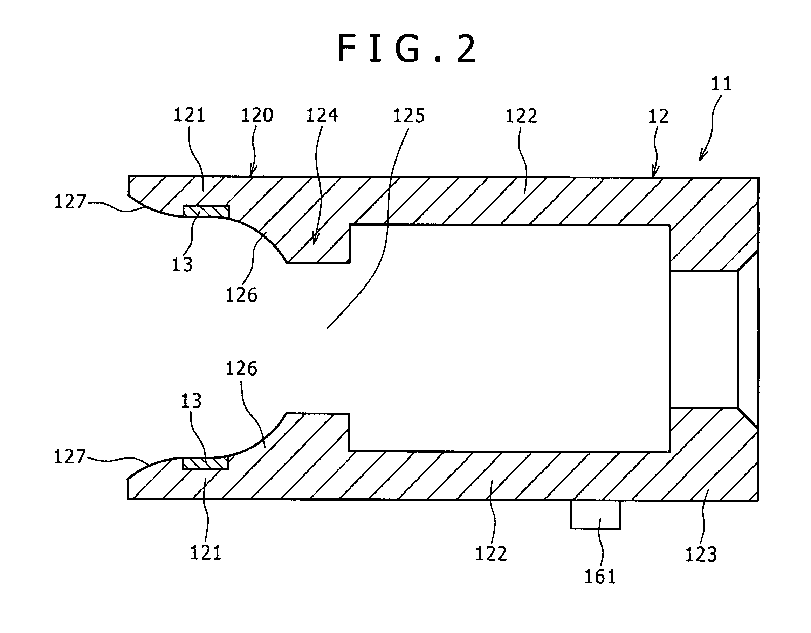

   FIG. 2 is a cross-sectional view illustrating needle puncture pain relieving means (a needle puncture pain relieving tool) of the puncture tool with mechanism for relieving needle puncture pain illustrated in FIG. 1.
[FIG. 3]

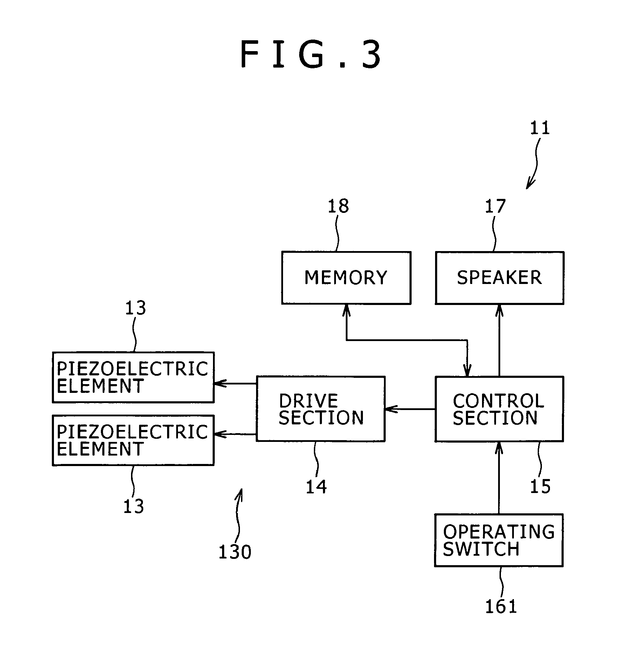

   FIG. 3 is a block diagram illustrating a circuit configuration of the needle puncture pain relieving means of the puncture tool with mechanism for relieving needle puncture pain illustrated in FIG. 1.
[FIG. 4]

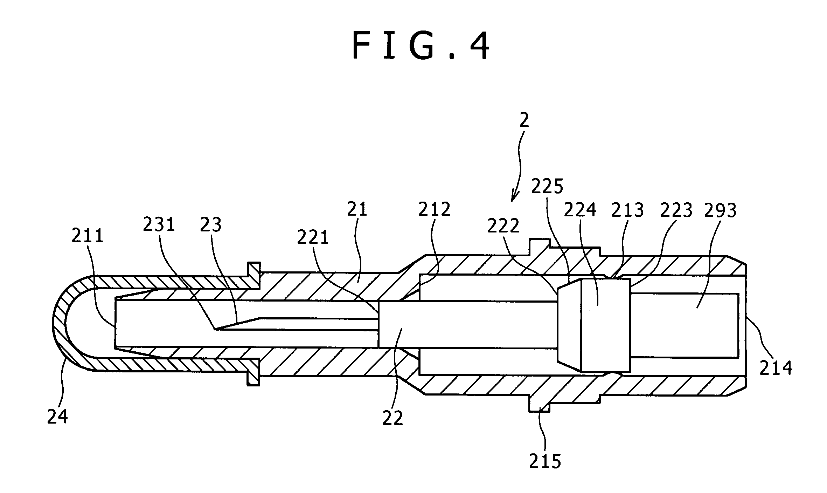

   FIG. 4 is a cross-sectional view illustrating a puncture needle cartridge attached to the puncture tool with mechanism for relieving needle puncture pain.
[FIG. 5]

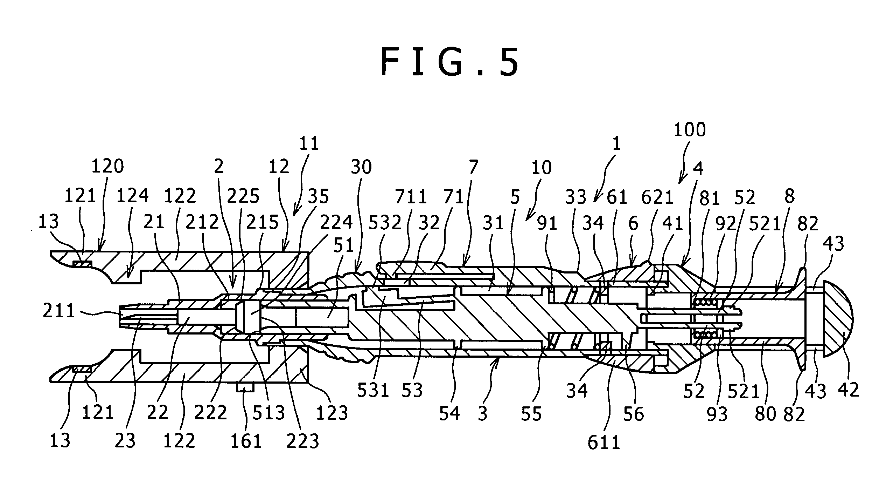

   FIG. 5 is a cross-sectional view illustrating a state of using the puncture tool with mechanism for relieving needle puncture pain illustrated in FIG. 1.
[FIG. 6]

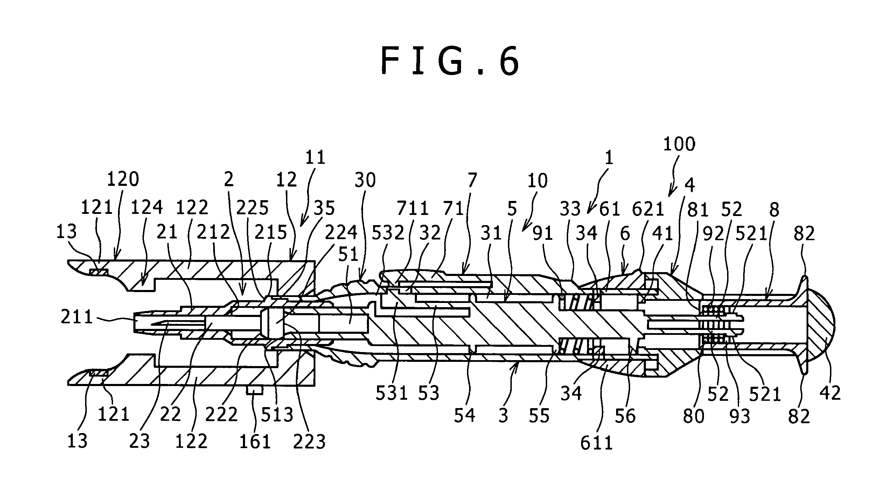

   FIG. 6 is a cross-sectional view illustrating a state of using the puncture tool with mechanism for relieving needle puncture pain illustrated in FIG. 1.
[FIG. 7]

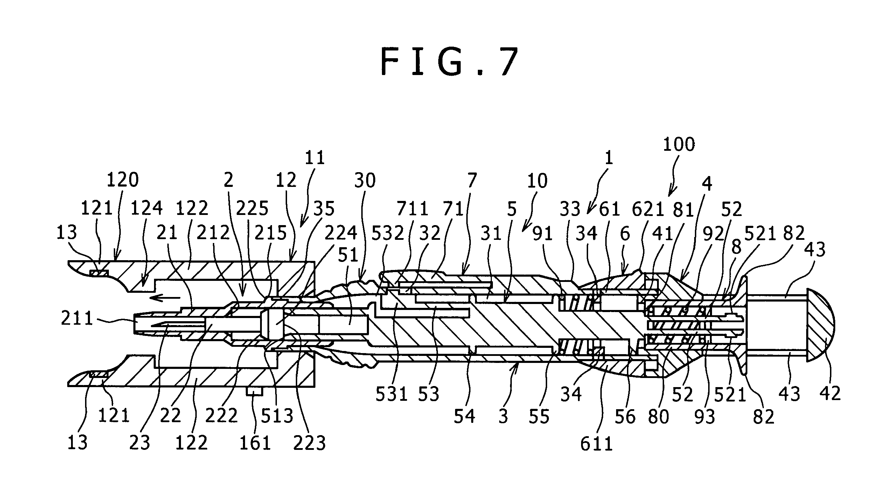

   FIG. 7 is a cross-sectional view illustrating a state of using the puncture tool with mechanism for relieving needle puncture pain illustrated in FIG. 1.
[FIG. 8]

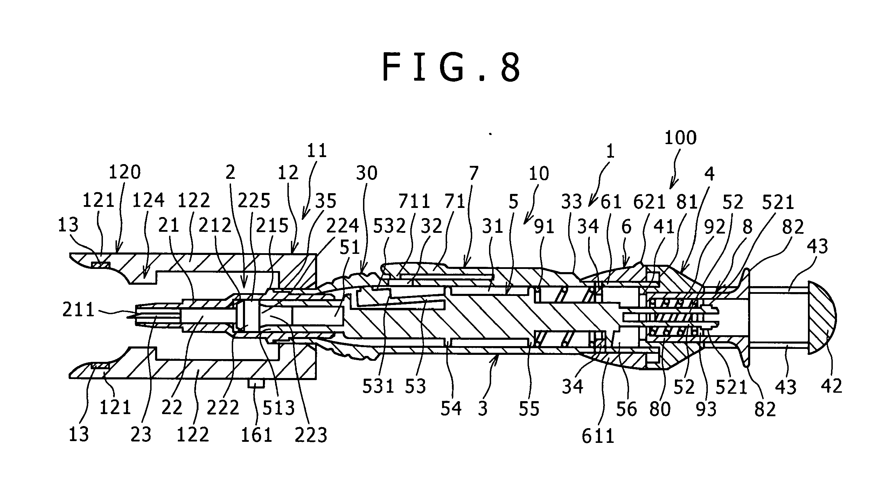

   FIG. 8 is a cross-sectional view illustrating a state of using the puncture tool with mechanism for relieving needle puncture pain illustrated in FIG. 1.
[FIG. 9]

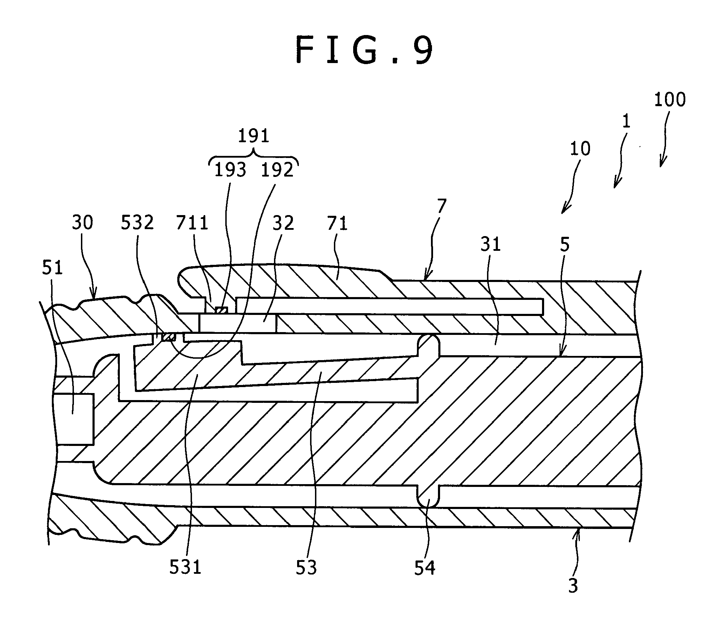

   FIG. 9 is a cross-sectional view illustrating a major portion of a second example of the puncture tool with mechanism for relieving needle puncture pain.
[FIG. 10]

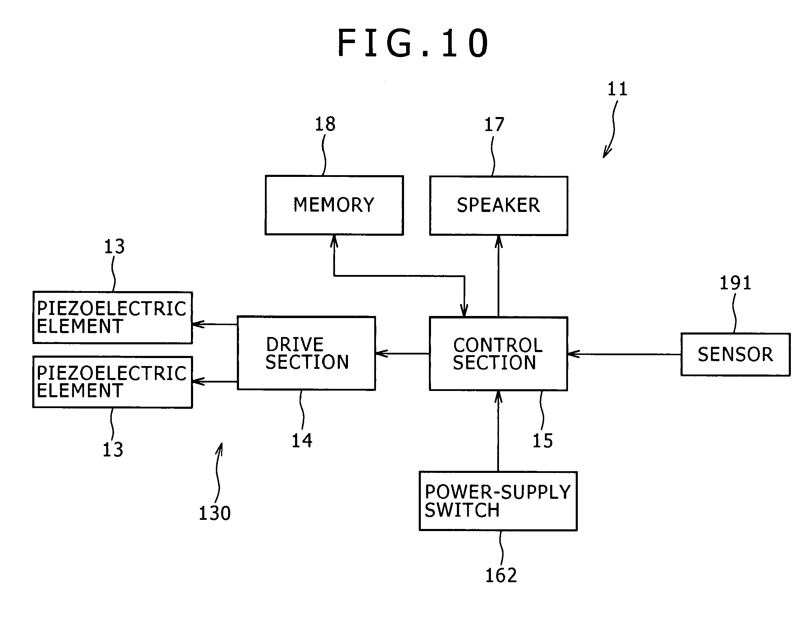

   FIG. 10 is a block diagram illustrating a circuit configuration of needle puncture pain relieving means of the puncture tool with mechanism for relieving needle puncture pain illustrated in FIG. 9.
[FIG. 11]

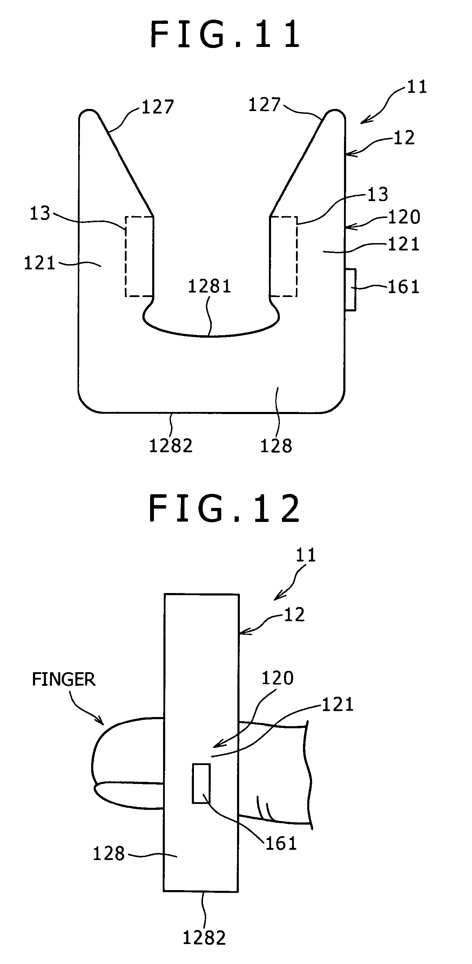

   FIG. 11 is a front view illustrating a third example of the needle puncture pain relieving tool according to the present invention.
[FIG. 12]
   FIG. 12 is a lateral view of the needle puncture pain relieving tool illustrated in FIG. 11.

### Best Mode for Carrying Out the Invention

A puncture tool with mechanism for relieving needle puncture pain and a needle puncture pain relieving tool according to the present invention will hereinafter be described in detail based on preferred embodiments in illustrated the accompanying drawings.

### <First Embodiment>

FIG. 1 is a cross-sectional view illustrating a first embodiment (the first embodiment of a needle puncture pain relieving tool and a puncture tool attached with the needle puncture pain relieving tool of the present invention) of a puncture tool with mechanism for relieving needle puncture pain of the present invention. FIG. 1(a) illustrates a state where a puncture needle cartridge is attached to the puncture tool with mechanism for relieving needle puncture pain (a state where the needle puncture pain relieving tool and the puncture needle cartridge are attached to the puncture tool). FIG. 1(b) illustrates a state where the needle puncture pain relieving means and a puncture needle cartridge are not attached to the puncture tool with mechanism for relieving needle puncture pain (a state where the needle puncture pain relieving tool and the puncture needle cartridge are not attached to the puncture tool). FIG. 2 is a cross-sectional view illustrating needle puncture pain relieving means (a needle puncture pain relieving tool) of the puncture tool with mechanism for relieving needle puncture pain illustrated in FIG. 1. FIG. 3 is a block diagram illustrating a circuit configuration of the needle puncture pain relieving means of the puncture tool with mechanism for relieving needle puncture pain illustrated in FIG. 1. FIG. 4 is a cross-sectional view illustrating the puncture needle cartridge attached to the puncture tool with mechanism for relieving needle puncture pain. FIGS. 5 to 8 illustrate states of using the puncture tool with mechanism for relieving needle puncture pain illustrated in FIG. 1.

Incidentally, a description is given taking a left-right direction (a longitudinal direction of a housing 3), a right side and a left side in FIGS. 1, 2 and 4 to 8 as "an axial direction," "a proximal end" and "a distal end," respectively.

As illustrated in these figures, a puncture tool 100 with mechanism for relieving needle puncture pain includes a puncture tool 1 and needle puncture pain relieving means (a needle puncture pain relieving tool) 11 detachably attached to the puncture tool 1. The needle puncture pain relieving tool 11 is a needle puncture pain relieving tool attached to the puncture tool 1 for use.

This puncture tool 100 with mechanism for relieving needle puncture pain (the puncture tool 1) is a tool which is used after a puncture needle cartridge 2 has been attached thereto and in which a puncture needle 23 of the puncture needle cartridge 2 punctures a surface (biological surface) of a fingertip (finger) pulp. The puncture needle cartridge 2 is detachably attached to a distal end portion of the puncture tool 1. After the front surface of the fingertip pulp has been punctured by the puncture needle 23, a necessary quantity of blood (body fluid) is collected and subjected to a blood test such as, blood glucose level measurement or the like.

The puncture tool 1 is first described.
As illustrated in FIGS. 1, the puncture tool 1 includes a puncture tool body 10 having an elongate housing 3; a plunger 5 to which the puncture needle cartridge 2 (the puncture needle 23) is detachably attached at a distal end portion; an adjusting portion 6; a puncture operating portion (operating means) 7; a set operating portion (operating portion) 8; a coil spring (first biasing means) 91 adapted to bias the plunger 5 in the distal end direction; and a coil spring (second biasing means) 92 adapted to bias the plunger 5 in the proximal end direction. A description is hereinafter given of the configurations of these components.

As illustrated in FIG. 4, the puncture needle cartridge 2 includes a tubular casing (puncture needle holder) 21; a lancet (puncture needle hub) 22 axially movably housed in the casing and provided with the puncture needle 23; and a cap 24 detachably attached to the distal end portion of the casing 21.

The distal end of the casing 21 is a region in contact with the front surface (biological surface) of a fingertip pulp and is formed with an opening 211. The casing 21 is formed with an opening 214 at a proximal end.

The casing 21 is formed on an inner circumferential surface with a pair of hub engaging portions 213, 213 capable of retaining a large-diameter portion 224 of the lancet 22 (only one of the hub engaging portions 213 is depicted in FIG. 4). Since an end face 223 of the large-diameter portion 224 of the lancet 22 is retained by the hub engaging portions 213, the lancet 22 can be inhibited from disengaging from the proximal end side of the casing 21.

Since an end face 222 of the lancet 22 comes into contact with the face 212 of the casing 21, a maximum projecting length (maximum projecting amount) of the puncture needle 23 from the opening 211 of the casing 21 is restricted.

The lancet 22 can axially be moved in the casing 21 between a position where the end face 222 comes into contact with the face 212 and a position where the end face 223 comes into contact with the hub engaging portion 213.

As illustrated in FIG. 1 (the same holds true for FIGS. 5 to 8), the housing 3 is composed of a housing body 30, and a cap-like member 4 provided at the proximal end portion of the housing body 30. The housing 3 has also a function as a grip portion serving when the puncture tool 1 is used.

The housing 3 has a hollow portion 31 with an opening distal end. A puncture mechanism is installed in the hollow portion 31. The puncture mechanism includes the plunger 5; the coil spring 91 biasing the plunger 5 toward the distal end; and the coil spring 92 biasing the plunger 5 toward the proximal end. The coil spring 91 constitutes drive means for moving (displacing) the plunger 5 (an attachment portion 51) from a waiting position (a delivery preparation position) to a puncture position.

The casing 21 of the puncture needle cartridge 2 is detachably attached to the distal end portion of the housing 3. That is to say, when the puncture needle cartridge 2 is attached to the puncture tool body 10, the proximal end portion of the casing 21 is fitted to the distal end portion of the housing body 30.

The housing body 30 is provided on the upper side in FIG. 1 with an elastically deformable plate-like puncture operating portion 7 and a marker 33. The puncture operating portion 7 is means for acting on a lock member 53 to operatively start the movement (displacement) of the plunger 5 (the attachment portion 51) from the waiting position to the puncture needle position. Incidentally, the marker 33 is disposed on the proximal end side of the puncture operating portion 7.

The puncture operating portion 7 is formed at the distal end portion with an operating button 71 having a projection 711 projecting downward in FIG. 1. The housing body 30 is formed at a position corresponding to the projection 711 with an opening 32 greater than the projection 711 and smaller than the operating button 71.

A pair of elongate holes 34, 34 is formed on the proximal end side of the mark 33 of the housing body 30 and along the circumferential direction thereof. One of the elongate holes 34, 34 is disposed on the upper side in FIG. 1 and the other on the lower side.

The plunger 5 is installed in the housing 3 so as to be movable (displaceable) in the axial direction. Specifically, the plunger 5 is configured to be movable (displaceable) between a waiting position (see FIGS. 6 and 7) and a puncture position (see FIG. 8) described later.

The plunger 5 is provided at the distal end portion with a cylindrical attachment portion (attachment means) 51 to which the lancet 22 of the puncture needle cartridge 2 is detachably attached. Specifically, when the puncture needle cartridge 2 is attached to the puncture tool body 10, the proximal end portion of the puncture needle 23 is fitted into the distal end portion of the attachment portion 51. In this case, a distal end 513 of the attachment portion 51 comes into contact with the end face 223 of the puncture needle 23.

The plunger 5 is provided at a proximal end portion with a first projecting portion 56 provided to extend downward in FIG. 1.

The plunger 5 is provided at the proximal end portion with a pair of rod-like projecting portions 52, 52 provided to extend toward the proximal end side. The projecting portions 52 are each formed with a projection 521 at the proximal end portion. One of the projecting portions 52 is disposed on the upper side in FIG. 1 and the other on the lower side.

The plunger 5 is provided with an elastically deformable rod-like lock member 53 on the upper side in FIG. 1. Incidentally, the lock member 53 is disposed on the proximal end side of the attachment portion 51.

The lock member 53 is formed at the distal end portion with a retaining portion 531 provided with a projection 532 projecting toward the upper side in FIG. 1.

In the state where puncture preparation (setting) described later has been completed, while the plunger 5 is biased by the coil spring 91 in the distal end direction relative to the housing 3, the projection 532 of the retaining portion 531 is inserted into the opening 32 of the housing body 30. Thus, the lock member 53 is retained with respect to the housing 3 (see FIG. 7). In this state, the projection 532 of the retaining portion 531 is not in contact with the projection 711 of the operating button 71. The position of the plunger 5 at this time is referred to as "the waiting position." The lock member 53 constitutes holding means for holding the plunger 5 (the attachment portion 51) at the waiting position.

The plunger 5 is formed with ringlike flanges 54 and 55 on the outer circumferential surface. The flange 55 is disposed closer to the proximal end side than the flange 54.

When the plunger 5 is moved, the flanges 54 and 55 slide along the inner circumferential surface of the housing body 30 to thereby hold the posture of the plunger 5.

A proximal end side portion of the flange 55 functions as a spring seat to which the distal end side of the coil spring 91 comes into contact. A tubular adjusting portion (adjusting dial) 6 is installed at the proximal end portion of the housing body 30 and on the distal end side of the cap-like member 4 so as to be turnable in the circumferential direction of the housing body 30.

The adjusting portion 6 is formed on the inner circumferential surface with a pair of puncture-depth control plates 61, 61. The puncture-depth control plates 61, 61 are each formed with a ringlike support portion (a second projecting portion) 611 which comes into contact with the projecting portion 56 of the plunger 5 when puncture is done. The pair of puncture-depth control plates 61, 61 is inserted into the pair of respective elongate holes 34, 34 of the housing body 30 described earlier and located in the housing body 30.

The puncture-depth control plates 61, 61 are arranged to face each other via the center of the support portion 611. The support portion 611 is disposed concentrically with the housing body 30 and at about the axial midpoint of the adjusting portion 6.

The puncture-depth control plate 61 has a portion where contact portions in contact with the projecting portion 56 of the plunger 5 are different from each other in axial position. This makes it possible to adjust the puncture depth (= an amount of projection of the lancet 22 from the opening 211) of the lancet 22 (the needle 231) into the biological surface.

As illustrated in FIG. 1, the set operating portion 8 (a set operating portion main body 80) is installed in the cap-like member 4 (on the proximal end side of the puncture body 10) so as to be movable in the axial direction. The set operating portion 8 is means for executing operation in which the plunger 5 (the attachment portion 51) is moved (displaced) in the proximal end direction and located at (returned to) the waiting position.

The cap-like member 4 is formed at the distal end with a ringlike rib 41 to which the distal end of the set operation portion 8 comes into contact.

The cap-like member 4 is formed at the proximal end with a head (position restricting means) 42. When the set operating portion 8 is operated, the proximal end of the set operation portion 8 comes into contact with the head 42 to inhibit the proximal end directional movement of the set operating portion 8.

The cap-like member 4 (the puncture tool body 10) is formed with an elongate hole (opening) 43 on each of the upper and lower sides in FIG. 1.

The set operating portion 8 has the cylindrical set operating portion main body 80 housing the coil spring 92.

The set operating portion main body 80 is formed with a ring-like rib 81 at the distal end. A proximal end side portion of the rib 81 functions as a spring seat to which the distal end side of the coil spring 92 comes into contact.

A finger-putting portion (a finger-laying portion) 82 is formed on each of the upper and lower sides, in FIG. 1, of the proximal end portion of the set operating portion main body 80 (the set operating portion 8). The finger-putting portion (a finger-laying portion) 82 is composed of a projecting piece projecting from the corresponding elongate hole 43 of the cap-like member 4. The finger-putting portion 82 on the upper side in FIG. 1 projects from the elongate 43 toward the upper side in FIG. 1. In addition, the finger-putting portion 82 on the lower side in FIG. 1 projects from the elongate 43 toward the lower side in FIG. 1. The finger-putting portions 82 are each moved in the elongate hole 43 along with the movement of the set operating portion main body 80.

A ringlike spring-receiving member 93 having a spring seat on the distal end side is installed in the set operating portion 8 so as to be movable in the axial direction.

If the puncture needle cartridge 2 is not attached to the puncture tool body 10, the coil spring 92 is inserted between the spring-receiving member 93 and the rib 81 while being substantially under no load (having a natural length). The coil spring 92 is located closer to the proximal end side than the coil spring 91. The spring constant of the coil spring 92 is smaller than that of the coil spring 91.

The spring-receiving member 93 is retained by a pair of projections 521, 521 while receiving a pair of projecting portions 52, 52 of the plunger 5 inserted thereinto.

Conditions such as the spring constant, axial lengths and the like of the coil springs 91 and 92 are set so that reliable puncture can be carried out. Specifically, when the puncture is carried out, the lancet 22 is surely projected at a given amount from the opening 211 of the casing 21 and thereafter surely housed in the casing 21.

The needle puncture pain relieving means (the needle puncture pain relieving tool) 11 is next described.
The needle puncture pain relieving means 11 is a tool that applies vibratory stimulations to a fingertip (finger) to relieve (reduce) a pain (puncture pain) occurring when the surface of the fingertip (the biological surface) pulp is punctured with the puncture needle 23 of the puncture needle cartridge 2. Its principle is as below.

The vibratory stimulations applied to the fingertip first reaches a brain from proper palmer digital nerves through median nerves and spinal marrow and are perceived as vibrations (vibratory sense) at a predetermined site of the brain. On the other hand, as with the vibratory stimulations mentioned above, also a wound occurring when the surface of the fingertip pulp is punctured with the puncture needle 23 reaches the brain from the proper palmer digital nerves through the median nerves and spinal marrow and is perceived as a pain (pain sense) at a predetermined site of the brain. A site of the brain perceiving a vibratory sense (pressure sense) is in very close to a site perceiving a pain sense. Therefore, the surface of the fingertip pulp may be punctured with the puncture needle 23 while the vibratory stimulations are applied to the fingertip. In such a case, because of processing the vibratory sense, the brain cannot sufficiently exhibit the cognition function of the pain sense. Thus, the processing of the pain sense becomes insufficient; in this way, the pain is relieved.

As illustrated in FIGS. 1 and 2, the needle puncture-pain relieving means 11 includes a main body portion 12 and a pair of piezoelectric elements (vibrators) 13, 13 installed in the main body portion 12.

The main body portion 12 includes a base portion (securing means) 123; a puncture portion 124 opposed to a fingertip (finger) pulp; a clip portion (contact portion) 120 having a pair of clip pieces 121, 121 disposed to face each other via the puncture portion 124 and coming into contact with respective opposite portions (lateral surfaces) of the fingertip (finger); and a pair of elastically deformable portions 122, 122 each connecting the base portion 123 with a corresponding one of the clip pieces 121.

The base portion 123 is formed circular (annular) and detachably attached to the distal end portion of the housing 3 of the puncture tool 1. Specifically, when the needle puncture pain relieving means 11 is attached to the puncture tool body 10 (the puncture tool 1), the distal end portion of the housing body 30 (housing 3) is fitted into (press fitted to) the base portion 123 from the proximal end side. In this way, the base portion 123 is secured to the puncture tool body 10 (the puncture tool 1). In other words, the needle puncture pain relieving means 11 is secured to the puncture tool 1. In this state, all portions other than the base portion 123 of the main body portion 12 are not in contact with any of the puncture tool 1 and the puncture needle cartridge 2. The central axis of the puncture needle cartridge 2 is aligned with that of the main body portion 12. In addition, the distal end portion of the casing 21 of the puncture needle cartridge 2 is located in a passage 125 of the puncture portion 124 described later.

The proximal end portion of the base portion 123 gradually increases in inner diameter as it goes in the proximal end direction. Thus, the needle puncture pain relieving means 11 can be attached to the puncture tool body 10 easily and rapidly.

The elastically deformable portions 122 are each formed like a rod or plate to have a longitudinal direction serving as an axial direction and arranged to face each other via the puncture needle cartridge 2 (the puncture needle 23). In this way, the puncture tool 1 is formed at the distal end portion with a recess portion for a finger used when the puncture needle cartridge 2 is attached to the distal end portion of the puncture tool 1. Thus, the puncture needle cartridge 2 can be attached easily and rapidly.

The outer surface of each of the elastically deformable portions 122 is formed as a plane stepless-continuous with the outer circumferential surface of the base portion 123 and the outer surface of the clip portion 120 described later.

The clip portion 120 is a portion fitted to a fingertip. The pair of piezoelectric elements 13, 13 is installed on the pair of respective clip pieces 121, 121 so that the vibrations of the piezoelectric elements 13 can apply vibratory stimulations to the opposite sides of a fingertip.

This can relieve a pain occurring when the surface of the fingertip pulp is punctured with the puncture needle 23 as described above.

The vibratory stimulations are applied to the opposite sides of the fingertip to relieve a pain. A portion to which vibrations are applied is apart in distance from a portion subjected to puncture compared with the case where vibratory stimulations are applied to the fingertip pulp. Therefore, the vibrations can be applied without giving a pain to a portion scarred when a previous measurement was done without giving a vibratory pain. In other words, the finger is elastically gripped and vibrated from the opposite sides thereof in a direction perpendicular to the puncture direction of the puncture needle 23; therefore, the effects described above can be produced.

The piezoelectric elements 13 are disposed on the corresponding surface sides of the clip pieces 121 in contact with the respective lateral portions of a fingertip. Further, in the configuration illustrated in the figure, each of the piezoelectric elements 13 is such that its surface on the lateral side of the fingertip is exposed to the outside. In this way, the piezoelectric elements 13 can directly apply vibratory stimulations to the lateral portions of the fingertip, i.e., can efficiently apply vibratory stimulations to the opposite sides of the fingertip.

Incidentally, the piezoelectric elements 13 which are vibrating bodies may be buried in the corresponding clip pieces 121 and may be disposed at respective portions (positions) of the clip pieces 121 different from those mentioned above.

A clearance distance between the pair of clip pieces 121, 121 is set to be smaller than the width of the fingertip. If the clip portion 120 is fitted to the fingertip (if the fingertip is located between the pair of clip pieces 121, 121), the pair of elastically deformable portions 122, 122 is elastically deformed. Its restoring force (resilience) allows the pair of clip pieces 121, 121 to grip and press the fingertip from the opposite sides thereof. In this way, the periphery of the puncture portion of the fingertip is squeezed to allow the puncture portion (wound) to bleed moderately. Thus, it is possible to collect a necessary quantity of blood easily and rapidly. Additionally, it becomes easy to transmit vibrations from the piezoelectric elements 13 to the corresponding sides of the fingertip. Thus, the vibratory stimulations can be applied to the opposite sides of the fingertip efficiently.

The front surface of the portion, of each of the clip piece 121, in contact with the fingertip has a curved concave surface corresponding to the shape of the fingertip. Thus, the clip pieces 121 can come into adequate contact with the corresponding lateral portions of the fingertip to grip it.

The opposite surfaces of the distal end portions of the clip pieces 121 are each formed with a guide surface (a guide portion) 127. The guide surfaces 127 are tilted so that the clearance distance between the pair of clip pieces 121, 121 is gradually increased as it goes in the distal end direction. Thus, the clip portion 120 can be fitted to the fingertip easily and rapidly.

It is preferred that the front surfaces of the portions, of the clip pieces 121, in contact with the lateral sides of the fingertip be each formed with a plurality of concavities and convexities (minute concavities and convexities). In this way, the clip pieces 121 come into point-contact with the lateral portions of the fingertip, thereby further relieving a pain.

It is preferred that the front surfaces of the portions, of the clip pieces 121, in contact with the lateral sides of the fingertip be each formed with a covering layer, not illustrated, made of a material having viscosity higher than that of the clip piece 121. This can improve adhesion between the clip piece 121 and the lateral portion of the fingertip. Therefore, it becomes easy for vibrations to be transmitted from the piezoelectric elements 13 to the corresponding lateral portions of the fingertip. Thus, vibratory stimulations can be applied to the opposite sides of the fingertip efficiently.

The puncture portion 124 has contact portions 126 in contact with the fingertip and a passage 125 adapted to receive the puncture needle 23 passed through there. A pair of the contact portions 126 is provided so as to correspond to the clip pieces 121, i.e., the contact portions 126 are provided at two respective positions. The contact portions 126 are each designed to come into contact with the vicinity (an end portion of the pulp and an end portion of the lateral portion) of a boundary portion between the pulp and lateral portion of the fingertip.

The front surface of each contact portion 126 has a curved concaved surface corresponding to the shape of the fingertip. Thus, the contact portions 126 can accurately come into contact with the fingertip.

The axial position of the proximal end of each contact portion 126 (curved concave surface) generally coincides with that of the opening 211 of the casing 21 of the puncture needle cartridge 2.

As illustrated in FIG. 3, the needle puncture pain relieving means 11 includes a vibrator 130, a control section (control means) 15, an operating switch 161, a speaker (information means) 17, a memory (storing means) 18, and a power supply part, not illustrated, adapted to supply electric power to the component parts. The vibrator 130 has a drive section 14 adapted to apply drive voltage (electric voltage) to the piezoelectric elements 13 to drive (vibrate) them. The vibrator 130, the control section 15, the operating switch 161, the speaker 17, a sensor 18 and the power supply part are installed on the main body portion 12. Incidentally, the piezoelectric elements 13 are each provided with an electrode not illustrated and the drive section 14 applies drive voltage to the piezoelectric elements via the corresponding electrodes.

The control section 15 is composed of e.g. a microcomputer, CPU or the like and controls the operation (driving) of the entire needle puncture pain relieving means 11, i.e., the vibrator 130, the speaker 17, the memory 18 and the like.

The memory 18 is composed of a semiconductor memory or the like such as e.g. ROM, a flash memory, EPROM, EEPROM or RAM. The memory 18 is adapted to store (record) various programs such as a program used to execute the control operation of e.g. the needle puncture pain relieving means 11, various date and the like. These programs and date are read out from the memory 18 when necessary.

The operating switch 161 is an operating section having a switch used to switch on/off of the vibration of each piezoelectric element 13. The operation of the operating switch 161 can actuate or stop the vibrator 130, i.e., start or stop the vibrations of the piezoelectric elements 13.

A description is next given of vibratory patterns (drive patterns) of each piezoelectric element 13. The vibratory patterns of the piezoelectric elements 13 are the same; therefore, one of the piezoelectric elements 13 is explained representatively.

As long as the vibratory frequency of the piezoelectric element 13 has such magnitude that a fingertip can perceive vibrations (that a pressure-receiving container can perceive vibrations), it is not restricted. However, 1000 Hz or below is preferable and approximately 30 to 300 Hz is more preferable. As long as the vibratory amplitude (peak-to-peak) of the piezoelectric element 13 has such magnitude that the fingertip can perceive the vibrations, it is not restricted. However, 6 µm or higher is preferable. As long as the vibratory width (a half value of a wavelength) of the piezoelectric element 13 is such a size that the fingertip can perceive vibrations, it is not restricted. However, 40 µm or higher is preferable.

In this way, vibratory stimulations with necessary and sufficient magnitude can be applied to a user's (patient's) fingertip safely and reliably.

The piezoelectric element 13 may be designed such that the level (intensity) of vibration varies with time or is constant. Incidentally, controlling the control section 15 can vary with time the vibratory level of the piezoelectric element 13.

Examples of configurations to vary with time the vibratory level of the piezoelectric element 13 include the following (1) and (2).

(1) A period for actuating the vibrator 130 and a period for halting it, i.e., a period for vibrating the piezoelectric element 13 (hereinafter, referred to as "the vibration-on period") and a period for not vibrating it (hereinafter, referred to as "the vibration-off period") are alternately provided.

In this case, the vibration-on period is a period where the vibration is "strong" and the vibration-off period is a period where the vibration is "weak."

In the vibration-on period, the frequency, amplitude, width and the like of the vibration of the piezoelectric element 13 may be varied with time to vary the level of the vibration with time.

Incidentally, the user executes puncture in the vibration-on period.

(2) Only the vibration-on period is provided and the frequency, amplitude, width and the like of the vibration of the piezoelectric element 13 is varied with time to vary the level of the vibration with time.

In order here to further relieve a pain occurring when the front surface of the fingertip pulp is punctured with the puncture needle 23, a user's sensation is made not to concentrate on the puncture (pain).

It is a preferable configuration that the speaker 17 delivers a message (sound) to prompt the user to count the number of variations of the vibratory level of the piezoelectric element 13. While the user counts the number of the variations of the vibratory level of the piezoelectric element 13, the user's sensation concentrates on it, so that the user lowers the ability to perceive a pain, further relieving the pain.

A description is next given of a method of (function of) using the puncture tool 100 with mechanism for relieving needle puncture pain (function).
[1] An unused puncture needle cartridge 2 is first attached to the distal end portion of the puncture tool 1 in the puncture tool 100 with mechanism for relieving needle puncture pain. Specifically, the proximal end portion of the casing 21 is fitted to the distal end portion of the housing 3 until a rib 215 of the casing 21 comes into contact with the distal end 35 of the housing 3. In addition, the proximal end portion of the lancet 22 is fitted to the distal end portion of the attachment portion 51 of the plunger 5.

In this case, as described above, the large-diameter portion 224 of the lancet 22 is retained by the hub engaging portions 213 of the casing 21. Therefore, the lancet 22 is not moved in the distal end direction unless a force equal to or greater than a predetermined value is applied. In addition, the flange 55 of the plunger 5 is in contact with the distal end of the coil spring 91.

Force adapted to move the large-diameter portion 224 of the lancet 22 from the hub engaging portions 213 is set to a level greater than the force compressing the coil springs 91, 92. Thus, the operation of fitting the proximal end portion of the lancet 22 to the distal end portion of the attachment portion 51 of the plunger 5 can simultaneously move the plunger 5 in the proximal end direction against the elastic force of the coil springs 91, 92. The projection 532 of the retaining portion 531 of the lock member 53 in the plunger 5 is inserted into the opening 32 of the housing 3. The plunger 5 is disposed at the waiting position, thus, completing the preparation (setting) of puncture. Further, the lancet 22 is pressed in the proximal end direction to move the large-diameter portion 224 of the lancet 22 from the hub engaging portions 213 and is released from the engagement with the hub engaging portions 213. The proximal end side surface of the rib (flange) 215 comes into contact with the distal end 35 of the housing body 30 so that the puncture needle cartridge 2 is stopped (see FIG. 7).

In this way, a to-be-attached portion 293 of the lancet 22 can reliably (easily) be fitted to the attachment portion 51 of the plunger 5. In addition, the preparation of puncture can be done easily and rapidly.

Incidentally, in the puncture body 10 not attached with the puncture needle cartridge 2, the projection 532 of the retaining portion 531 comes into contact with the inner circumferential surface of the housing 3 so that the lock member 53 of the plunger 5 is displaced (elastically deformed) downwardly in FIG. 1(b). In short, the retaining portion 531 is biased upward in FIG. 1(b) by the elastic force of the lock member 53.

The attachment of the puncture needle cartridge 2 may be done after the removal of the cap 24 from the puncture needle cartridge 2. However, it is preferred that the attachment of the puncture needle cartridge 2 be done with cap 24 attached. It is more preferred that the attachment of the puncture needle cartridge 2 be done while pressing the cap 24 toward the proximal end side.

If the attachment of the puncture needle cartridge 2 is done with the cap 24 attached, the cap 24 is removed from the puncture needle cartridge 2 after the attachment of the puncture needle cartridge 2, preferably immediately before puncture.

[2] When the puncture needle cartridge 2 is attached to the distal end portion of the puncture tool 1, force adapted to shift the large-diameter portion 224 of the lancet 22 from the hub engaging portion may be set to such a degree that the plunger 5 is not shifted to the waiting position. In such a case, after the puncture needle cartridge 2 has been attached to the distal end portion of the puncture tool 1, the following operation can dispose the plunger 5 at the waiting position for the preparation (setting) of the puncture.

As illustrated in FIGS. 5 and 6, fingers are put (laid) on the finger-putting portion 82 of the set operating portion 8 and the set operating portion 8 is shifted in the proximal end direction with respect to the housing 3 against the resilient force of the coil springs 91 and 92. When the proximal end (proximal end face) of the set operating portion 8 comes into contact with the head 42 (a distal end face 421), the finger is released from the finger-putting portion 82.

In this case, as illustrated in FIG. 5, the coil spring 92 with a small spring constant is first compressed and as illustrated in FIG. 6 the coil spring 91 with a large spring constant is next compressed. Thus, in accordance with the compression of the coil spring 91, the plunger 5 is shifted in the proximal end direction.

As illustrated in FIG. 6, when the set operating portion 8 is shifted until its proximal end comes into contact with the head 42 of the housing 3, the projection 532 of the retaining portion 531 of the lock member 53 of the plunger 5 is located at the position of the opening 32 of the housing 3. The lock member 53 having elastically been deformed is restored to an original shape due to its resilient force, so that the projection 532 is inserted into the opening 32 of the housing 3 and opposed to the projection 711 of the operating button 71.

When the fingers are released from the finger-putting portion 82, as illustrated in FIG. 7, the projection 532 of the retaining portion 531 comes into contact with the distal end side edge portion facing the opening 32 of the housing 3. Thus, the retaining portion 531 is retained (locked) with respect to the housing 3. In this way, the compressed state of the coil spring 91, i.e., the state where the plunger 5 is biased in the distal end direction relative to the housing 3 by the coil spring 91 is kept so that the plunger 5 is set at the waiting position.

On the other hand, when the fingers are released from the finger-putting portion 82, as illustrated in FIG. 7, the coil spring 92 having been compressed (in the compressed state) is extended by its resilient force. Thus, the set operating portion 8 is shifted toward the distal end side until the rib 81 comes into contact with the rib 41 of the housing 3. In addition, the coil spring 92 is restored to a natural length so that it biases the plunger 5 in neither of the distal and proximal end directions.

In this state, the preparation (setting) for puncture of the puncture needle 23 into the biological surface is completed. The operation of the above-mentioned [3] allows for re-puncture of the puncture tool in a non-attached state.

[3] The puncture depth of the lancet 22 (the needle tip 231) into the biological surface is adjusted as necessary. Specifically, the puncture depth of the lancet 22 into the biological surface is set to one according to the individual difference of a blood-collected person or to a puncture site.

A criterion used to set the puncture depth can be set to a puncture depth where a minimal amount of blood necessary for e.g. blood glucose measurement can be obtained. This can suppress a puncture pain to a minimal level.

If the puncture depth is set once, it is not necessary to reset the puncture depth every time blood is collected. Thus, the operation is not cumbersome.

[4] The operating switch 161 is operated to start the vibrations of the piezoelectric elements 13. In addition, the clip portion 120 of the puncture tool 100 with mechanism for relieving needle puncture pain is fitted to a fingertip from the pulp side thereof. In this way, the pair of clip pieces 121, 121 grips the fingertip from the opposite sides thereof. The pair of contact portions 126 is press fitted to the front surface of the fingertip in the vicinity of the boundary portion between the pulp and each lateral portion thereof. In addition, the distal end of the casing 21 of the puncture needle cartridge 2 is press fitted to the surface of the fingertip pulp. Incidentally, of the surface of the fingertip pulp, a site in the vicinity of the distal end of the casing 21 is not in contact with the main body portion 12 because of the passage 125 or the like.

Next, the operating button 71 of the puncture operating portion 7 is next pressed (depressed downward in FIG. 7).
If the operating button 71 is pressed, as illustrated in FIG. 8, the projection 711 presses the projection 532 of the retaining portion 531. In this way, the lock member 53 is elastically deformed to displace the retaining portion 531 downward in FIG. 8, so that the engagement the projection 532 of the retaining portion 531 with the edge portion facing the opening 32 is disengaged (released).

On the other hand, because the puncture operating portion 7 is elastically deformed, when the finger is released from the operating button 71, the puncture operating portion 7 having elastically been deformed is restored to the original shape due to the resilient force thereof.

If the engagement of the retaining portion 531 is released, as illustrated in FIG. 8, the coil spring 91 having been compressed is extended due to the resilient force thereof to shift the plunger 5 in the distal end direction. Thus, the puncture needle 23 is projected from the opening 211 of the casing 21 to puncture the biological surface. At this time, the plunger 5 is shifted (displaced) from the waiting position to the puncture position.

When the plunger 5 is shifted in the distal end direction, along the way, the coil spring 91 and the flange 55 are moved away from each other and the coil spring 92 is compressed. Thus, the plunger 5 is biased by the coil spring 92 in the proximal end direction with respect to the housing 3.

In this case, the spring constant of the coil spring 91 is greater than that of the coil spring 92 as described above. Therefore, the plunger 5 is shifted toward the distal end side against the resilient force of the coil spring 92 so that the puncture needle 23 can puncture the biological surface.

After moving away from the flange 55, the coil spring 91 is restored to the natural length so that it biases the plunger 5 in neither of the distal and proximal end directions.

After the puncture needle 23 punctured the surface of the fingertip pulp, the coil spring 92 having been compressed is extended by the resilient force thereof as illustrated in FIG. 1(a). The plunger 5 is shifted in the proximal end direction so that the puncture needle 23 comes out of the surface of the fingertip pulp and is housed (stored) in the casing 21. In other words, the puncture needle 23 is returned to the state illustrated in FIG. 1(a). As described above, the puncture needle 23 is designed not to project from the opening 211 of the casing 21 at times other than the puncture. Thus, the puncture needle will not accidentally damage skin or the like to prevent infection, which provides satisfactory safety.

Additionally, the opposite sides of the fingertip are subjected to the vibrations of the piezoelectric elements 13; therefore, a pain occurring when the surface of the fingertip pulp is punctured with the puncture needle 23 is relieved.

After the puncture, the state where the opposite sides of the fingertip are subjected to the vibratory stimulations is kept (maintained) for a predetermined period of time. Therefore, also a pain (secondary pain) after the puncture can be relieved.

[5] The puncture tool 100 with mechanism for relieving needle puncture pain is removed from the fingertip. The operating switch 161 is operated to stop the vibrations of the piezoelectric elements 13. Blood on the puncture site is collected. The collection of blood can directly supply blood onto test paper or suck blood via a narrow tube and supply it to the test paper, for example.

As described above, the puncture tool 100 with mechanism for reliving needle puncture pain can relieve a pain occurring when the surface of the fingertip (finger) pulp is punctured with the puncture needle 23 of the puncture cartridge 2.

That is to say, because of having the needle puncture pain relieving means 11, the pain occurring when the surface of the fingertip pulp is punctured with the puncture needle 23. In particular, the pain is relieved by applying the vibratory stimulations to the opposite sides of the fingertip. Therefore, the pain can be relieved reliably and largely compared with the case where the pain is relieved by applying the vibratory stimulations to the fingertip pulp.

The piezoelectric elements 13 are installed on the respective clip pieces 121 coming into contact with the corresponding opposite sides of the fingertip. The pain is relieved by applying the vibratory stimulations to the opposite sides of the fingertip. Therefore, it is possible to prevent the increase of the pain of the scar (puncture scar) formed by the previous puncture.

Incidentally, the needle puncture pain relieving means 11 is configured to be detachably attached to the puncture tool 1 in the present embodiment. However, the invention is not limited to this. For example, the needle puncture pain relieving means 11 may be secured to (fixedly installed on) the puncture tool 1.

The piezoelectric element 13 is installed on each of the pair of respective clip pieces 121, 121 in the present embodiment. However, in the present invention, the piezoelectric element 13 is installed in either of the pair of clip pieces 121, 121.

In the present embodiment, the piezoelectric element 13 is used as the vibrating body. However, the present invention is not limited to this. For example, a vibratory motor or the like may be used as the vibrating body (vibrator).

### <Second Example>

FIG. 9 is a cross-sectional view illustrating a major portion of a second example of the puncture tool with mechanism for reliving needle puncture pain according to the present invention. FIG. 10 is a block diagram illustrating a circuit configuration of needle puncture pain relieving means of the puncture tool with mechanism for relieving needle puncture pain illustrated in FIG. 9. Incidentally, a description is given with the right and left in FIG. 9 taken as "the proximal end" and "the distal end," respectively.

A description is hereinafter given of a second example of a puncture tool with mechanism for relieving needle puncture pain according to the present invention. However, points different from the first embodiment described above are mainly explained and the explanations of the same items are omitted.

The second embodiment is different from the first embodiment in the following. Needle puncture pain relieving means 11 includes detection means for detecting a predetermined state of a puncture tool with mechanism for relieving needle puncture pain. A control section 15 is configured to actuate and stop a vibrator 130. The needle puncture pain means 11 is secured to (fixedly installed on) the puncture tool 1.

As illustrated in FIGS. 9 and 10, in the puncture tool with mechanism for relieving needle puncture pain of the second example, the needle puncture relieving means 11 includes a sensor (detection means) 191 and a power-supply switch 162. The sensor 191 includes an electrode 192 installed on a projection 532 of a retaining portion 531 of a lock member 53 and a electrode 193 installed on a projection 711 of an operating button 71 of a puncture operating portion 7. The electrode 192 is disposed on the upper side of a projection 532 in FIG. 9 so that an upper surface of the electrode 192 is exposed to the outside. The electrode 193 is disposed on the lower side of the projection 711 in FIG. 9 so that the lower surface of the electrode 193 is exposed to the outside.

In the state where preparation (setting) of puncture is completed, i.e., in the state where the projection 532 of the retaining portion 531 faces the projection 711 of the operating button 71, the electrode 192 and the electrode 193 are disposed to face each other. In addition, in the state where the preparation of puncture is completed, the projection 532 of the retaining portion 531 is not in contact with the projection 711 of the operating button 71. In other words, the electrode 192 is not in contact with the electrode 193.

The sensor 191 is electrically connected to a control section 15. A signal (detection result) from the sensor 191 is supplied to the control section 15. Specifically, the control section 15 receives different signals between a case where the electrode 192 is not in contact with the electrode 193 and a case where the electrode 192 is in contact with the electrode 193. In this way, the control section 15 can determine whether or not the projection 532 of the retaining portion 531 is in contact with the projection 711 of the operating button 71.

The control section 15 exercises the following control on the basis of signals from the sensor 191.
In the state where the preparation (setting) of puncture is completed, if the operating button 71 of the puncture operating portion 7 is pressed, the electrode 192 and the electrode 193 come into contact with each other. In this way, it is detected that the operating button 71 of the puncture operating portion 7 is pressed, i.e., that operation of starting the displacement (shifting) of the plunger 5 from the waiting position to the puncture position is done. Immediately thereafter, the control section 15 starts the vibrations of the piezoelectric elements 13 and also starts measurement of time. The vibrations of the piezoelectric elements 13 apply vibratory stimulations to the opposite sides of a fingertip to relieve a pain occurring when the surface of the fingertip pulp is punctured with the puncture needle 23.

The control section 15 stops the vibrations of the piezoelectric elements 13 when the measurement period reaches a preset reference period. If the reference period is longer than a period where the puncture is ended, it is not particularly restricted. However, the reference period is preferably 10 seconds or less, more preferably approximately 2 to 6 seconds.

The reference period is set so that the vibrations of the piezoelectric elements 13 (the state where the vibratory stimulations are applied to the opposite sides of the fingertip) continue for a predetermined time after the puncture. Therefore, a pain after the puncture (secondary pain) can be relieved.

As described above, the puncture tool 100 with mechanism for relieving needle puncture pain provides the same effects as those of the first embodiment described above.

In the puncture tool 100 with mechanism for relieving needle puncture pain, the start and stop of the vibrations of the piezoelectric elements 13 are automatically done; therefore, operation is facilitated and convenience is improved.

Incidentally, in the present example, the needle puncture pain relieving means 11 is secured to the puncture tool 1. The needle puncture pain relieving means 11 or its major portion (e.g. a portion other than the sensor 191) may be configured to be detachably attached to the puncture tool 1. For example, the portion (the main body portion 12 and the like) of the needle puncture pain relieving means 11 other than the sensor 191 may be detachably attached to the puncture tool 1. In such a configuration, a first connector electrically connected to the sensor 191 is provided on the housing 3. In addition, a second connector electrically connected the control section 15 is provided on the main body portion 12. When the puncture tool body 10 (the puncture tool 1) is attached to the main body portion 12, the first and second connectors are connected to each other.

In the present example, the timing of starting the vibrations of the piezoelectric element 13 can include the time when the clip portion 120 is fitted to a fingertip and the time when the preparation (setting) of puncture is completed.

### <Third Example>

FIG. 11 is a front view illustrating a third example of a needle puncture pain relieving tool. FIG. 12 is a lateral view of the needle puncture pain relieving tool illustrated in FIG. 11. Incidentally, FIG. 12 illustrates the state where the clip portion of the needle puncture pain relieving tool is fitted to the fingertip.

A description is next given of a third example of a needle puncture pain relieving tool. However, points different from the first embodiment described above are mainly explained and the explanations of the same items are omitted.

The puncture pain relieving tool 11 illustrated in these figures is a tool used together with the puncture tool 1 (see FIG. 1) described above.

As illustrated in FIGS. 11 and 12, the needle puncture pain relieving tool 11 includes a main body portion 12 and a pair of piezoelectric elements (vibrating bodies) 13, 13 installed on the main body portion (casing) 12.

The main body portion 12 includes a bottom portion 128 and a clip portion (contact portion) 120 having a pair of clip pieces 121, 121. The clip pieces 121, 121 are disposed to face each other via the bottom portion 128 and come into contact with the corresponding opposite sides (lateral surfaces) of a fingertip (finger).

The main body portion 12 has a space defined when the finger is gripped by the pair of clip pieces 121, 121, the space being close to a site where the finger is punctured. In addition, the main body portion 12 has the bottom portion 128 at a portion different from the portion where the finger is punctured. The puncture can be done through the space; therefore, the puncture direction and the gripping direction are perpendicularly away from each other. The portion different from the portion where the finger is punctured includes a portion on the distal end side of the finger as well as a portion on the back of the portion where the finger is punctured, as illustrated in FIG. 11.

The grip portion 120 is a portion to which the fingertip is fitted (a portion adapted to receive the fingertip fitted thereto). If the clip portion 120 is fitted to the fingertip from the back side of the fingertip, the back of the fingertip comes into contact with an upper surface (a surface on the upper side in FIG. 11) 1281 of the bottom portion 128. The upper surface 1281 of the bottom portion 128 has a curved concave surface corresponding to the shape of the back of the fingertip. In this way, the fingertip can stably be fitted to the clip portion 120.

A lower surface (a surface on the lower side in FIG. 11) 1282 of the bottom portion 128 is flat. Thus, the needle puncture pain relieving tool 11 can stably be put on a flat surface.

A pair of piezoelectric elements 13, 13 is installed in the pair of respective clip pieces 121, 121 of the clip portion 120. The piezoelectric elements 13, 13 are vibrated to apply vibratory stimulations to the opposite sides of the fingertip.

In this way, as described above, the pain occurring when the surface of the fingertip pulp is punctured with the puncture needle 23 can be relieved.

Applying the vibratory stimulations to the opposite sides of the fingertip can relieve the pain. Compared with the case where vibratory stimulations are applied to the fingertip pulp, a portion to which vibrations are applied is apart in distance from a portion subjected to puncture. Thus, the vibrations can be applied without giving a pain resulting from the vibrations to the portion scarred when a previous measurement was done.

The piezoelectric elements 13 are each disposed on a surface side, of the corresponding clip piece 121, in contact with the lateral portion of the fingertip. Further, in the configuration illustrated in the figure, the piezoelectric elements 13 are each such that a surface on the lateral side of the fingertip is exposed to the outside. In this way, the piezoelectric elements 13 can directly apply vibratory stimulations to the lateral portions of the fingertip, i.e., efficiently apply the vibratory stimulations to the opposite sides of the fingertip.

Incidentally, the piezoelectric elements 13 which are vibrating bodies may be buried in the corresponding clip pieces 121 and may be disposed at respective portions (positions) of the clip pieces 121 different from those mentioned above, or at the bottom portion 128. In other word, the vibratory stimulations resulting from the vibrations of the piezoelectric element 13 need only to be applied to the lateral portion of the fingertip.

A clearance distance between the pair of clip pieces 121, 121 is set to be slightly smaller than the width of the fingertip. If the clip portion 120 is fitted to the fingertip (if the fingertip is located between the pair of clip pieces 121, 121), the pair of clip pieces 121, 121 grip and press the fingertip from the opposite sides thereof. In this way, the periphery of the puncture portion of the fingertip is squeezed to allow the puncture portion (wound) to bleed moderately. Thus, it is possible to collect a necessary quantity of blood easily and rapidly. Additionally, it becomes easy to transmit vibrations from the piezoelectric elements 13 to the corresponding lateral portions of the fingertip. Thus, vibrator stimulations can be applied to the opposite sides of the fingertip efficiently.

Preferably, a portion, of each clip piece 121, in contact with the fingertip has on its front surface a curved concave surface corresponding to the shape of the fingertip. Thus, the clip pieces 121 can come into accurate contact with the corresponding lateral portions of the fingertip to grip the fingertip.

The opposite surfaces of the distal end portions of the clip pieces 121 are each formed with a guide surface (a guide portion) 127. The guide surfaces 127 are tilted so that the clearance distance between the pair of clip pieces 121, 121 is gradually increased as it goes toward the upside in FIG. 11. Thus, the clip portion 120 can be fitted to the fingertip easily and rapidly.

It is preferred that the front surfaces of the portion, of the clip pieces 121, in contact with the lateral sides of the fingertip be each formed with a plurality of concavities and convexities (minute concavities and convexities). In this way, the clip pieces 121 come into point-contact with the lateral portions of the fingertip, thereby further relieving a pain.

It is preferred that the front surfaces of the portions, of the clip pieces 121, in contact with the lateral sides of the fingertip be each formed with a covering layer, not illustrated, made of a material having viscosity higher than that of the clip piece 121. This can improve adhesion between the clip piece 121 and the lateral portion of the fingertip. Therefore, it becomes easy for vibrations to be transmitted from the piezoelectric elements 13 to the corresponding lateral portions of the fingertip. Thus, vibratory stimulations can be applied to the opposite sides of the fingertip efficiently.

As illustrated in FIG. 3, the needle puncture pain relieving tool 11 includes a vibrator 130, a control section (control means) 15, an operating switch 161, a speaker (information means) 17, a memory (storing means) 18, and a power supply part, not illustrated, adapted to supply electric power to the component parts. The vibrator 130 has a drive section 14 adapted to apply drive voltage (electric voltage) to the piezoelectric elements 13 to drive (vibrate) them. The vibrator 130, the control section 15, the operating switch 161, the speaker 17, a sensor 18 and the power supply part are installed in the main body portion 12. Incidentally, in the present example, the drive section 14, the control section 15, the speaker 17, the memory 18 and the power supply part are installed on the bottom portion 128. The operating switch 161 is installed on the external surface on the side (on the root side) of the bottom portion 128 of one of the clip pieces 121. Incidentally, the piezoelectric elements 13 are each provided with an electrode not illustrated and the drive section 14 applies drive voltage to the piezoelectric elements via the corresponding electrodes.

A description is next given of an example method of (operation of) using the needle puncture pain relieving tool 11.

When blood is collected by the needle puncture pain relieving tool 11 puncturing the front surface of the fingertip pulp, the clip portion 120 of the needle puncture pain relieving tool 11 is fitted to the fingertip from the back side thereof. In this way, the pair of clip pieces 121, 121 grips the fingertip from the opposite sides thereof and the back of the fingertip comes into contact with the bottom portion 128.

The operating switch 161 is operated to start the vibrations of the piezoelectric elements 13. In this way, the vibrations of the piezoelectric elements 13 apply the vibratory stimulations to the opposite sides of the fingertip.

Next, the surface of the fingertip pulp is punctured with the puncture tool 1. At this time, the vibratory stimulations relieve the pain occurring when the surface of the fingertip pulp is punctured with the puncture needle 23. After the puncture, the state where the opposite sides of the fingertip are subjected to the vibratory stimulations is kept (maintained) for a predetermined period of time. Therefore, also a pain (secondary pain) after the puncture can be relieved.

Next, the operating switch 161 is operated to stop the vibrations of the piezoelectric elements 13. The needle puncture pain relieving tool 11 is removed from the fingertip. The operating switch 161 is operated to stop the vibrations of the piezoelectric elements 13. Blood on the puncture site is collected. The collection of blood can directly supply blood onto test paper or suck blood via a narrow tube and supply it to the test paper, for example.

As described above, the needle puncture pain relieving tool 11 can produce the same effects as those of the first embodiment described above.

### <Experimental Example>

In order to confirm the effects of the puncture tool 100 with mechanism for relieving needle puncture pain, the following experiment was performed on ten healthy subjects.

The puncture tool 100 with mechanism for relieving needle puncture pain according to the first embodiment was prepared and the clip portion 120 of the puncture tool 100 was fitted to a fingertip of a left index finger (second finger). While continuously giving an instantaneous pain (corresponding to a puncture pain) to the fingertip pulp, the piezoelectric elements 13 are vibrated to apply vibratory stimulations to the opposite sides of the fingertip.

The results show that six feel the pain being dissipated and four feel the pain being relieved.

In order to confirm the effects of the needle puncture pain relieving tool 11, the following experiment was performed on ten healthy subjects.

The tool 11 for relieving needle puncture pain according to the third example was prepared and the clip portion 120 of the tool 11 for relieving needle puncture pain was fitted to a fingertip of a left index finger (second finger). While continuously giving an instantaneous pain (corresponding to a puncture pain) to the fingertip pulp, the piezoelectric elements 13 are vibrated to apply vibratory stimulations to the opposite sides of the fingertip.

The results show that six feel the pain being dissipated and four feel the pain being relieved.

The puncture tool with mechanism for relieving needle puncture pain and the tool for relieving needle puncture pain have been described thus far on the basis of the first embodiment and the examples illustrated in the figures. The components constituting each of the puncture tool with mechanism for relieving needle puncture pain and the tool for relieving needle puncture pain can be replaced with corresponding components arbitrarily configured to exhibit the same functions. An arbitrarily configuration(s) may be added to them.

The embodiments in the case of collecting blood are described above; however, the puncture tool with mechanism for relieving needle puncture pain and the puncture tool may be used to collect body fluid such as e.g. intercellular fluid other than blood. Their use applications are not restricted.

The above describes the case of puncturing the finger pulp. However, if the lateral portion of a finger is punctured, "the finger pulp" and "the lateral portion of the finger" are read as "the lateral portion of the finger" and "the pulp or back of the finger," respectively. Specifically, if the surface of the lateral portion of the finger is punctured with the puncture needle, the needle puncture pain relieving tool of the present invention is a tool for relieving needle puncture pain that is used together with the puncture tool in which the surface of the lateral portion of the finger is punctured with the puncture needle and that relieves a pain occurring when the surface of the lateral portion of the finger is punctured with the puncture needle. In addition, the tool for relieving needle puncture pain includes the contact portion in contact with each of the pulp and back of the finger and the vibrator having a vibrating body applying vibratory stimulations to at least one of the pulp and back of the finger and driving the vibrating body.

### Industrial Applicability

The present invention can relieve (reduce) a pain occurring when the surface of a finger pulp is punctured with a puncture needle. That is to say, the pain occurring when the surface of the finger pulp is punctured with the puncture needle and the pain can be relieved by applying vibratory stimulations to the lateral portion of the finger. Therefore, it is possible to prevent the increase of the pain of a wound (puncture wound) formed on the surface of the finger pulp due to the previous puncture. Thus, the present invention has industrial applicability.

## Claims

1. A needle puncture pain relieving tool (11) for use with a puncture tool to puncture a surface of a finger pulp with a puncture needle to relieve a pain occurring when the surface of the finger pulp is punctured with the puncture needle, said tool comprising
a contact portion (126) having a vibrating body (13) and a pair of clip pieces (121, 121) being able to come into contact with opposite sides of a finger,
a vibrator (130) driving the vibrating body (13), and
a control section (15) adapted to control an operation of the vibrator (130) and to vary a level of the vibratory stimulations of the vibrating body (13) with time.

2. The needle puncture pain relieving tool (11) according to claim 1,
wherein
the clip pieces (121, 121) are disposed to face each other, and wherein
the vibrating body (13) is provided on at least one of the clip pieces (121, 121).

3. The needle puncture pain relieving tool (11) according to claim 1 or 2, wherein
the vibrating body (13) is disposed on a surface side of the clip piece (121) so as to be able to come into contact with a lateral portion of a finger.

4. The needle puncture pain relieving tool (11) according to any one of claims 1 to 3, wherein
the vibrating body (13) is provided on each of the clip pieces (121, 121).

5. The needle puncture pain relieving tool (11) according to any one of claims 1 to 4, wherein
a portion of the clip piece (121) being able to come into contact with a finger has on a front surface thereof a curved concave surface corresponding to a shape of a finger.

6. The needle puncture pain relieving tool (11) according to any one of claims 1 to 5, wherein
a portion of the clip piece (121) being able to come into contact with a lateral portion of a finger is formed with concavities and convexities on a front surface.

7. A puncture device (100) including a puncture tool (1) with a puncture portion (124) and a puncture needle (23), and the needle puncture pain relieving tool (11) according to one of the preceding claims, wherein
the contact portion (120) of the needle puncture pain relieving tool (11) has a pair of clip pieces (121, 121) oppositely disposed via the puncture portion (124).

## Patentansprüche

1. Hilfsmittel zur Linderung eines Nadeleinstichschmerzes (11) zur Verwendung mit einem Einstichhilfsmittel zum Einstechen einer Oberfläche einer Fingerkuppe mit einer Einstichnadel, um einen Schmerz zu lindern, der auftritt, wenn die Oberfläche der Fingerkuppe mit der Einstichnadel eingestochen wird, wobei das Hilfsmittel aufweist
einen Berührungsabschnitt (126) mit einem Vibrationskörper (13) und einem Paar Klammerstücke (121, 121), die in der Lage sind, entgegengesetzte Seiten eines Fingers zu berühren,
einen Vibrator (130), der den Vibrationskörper (13) antreibt, und einen Steuerungsteil (15), der angepasst ist, um einen Betrieb des Vibrators (130) zu steuern und um eine Stufe der vibrierenden Stimulationen des Vibrationskörpers (13) mit der Zeit zu variieren.

2. Hilfsmittel zur Linderung eines Nadeleinstichschmerzes (11) nach Anspruch 1, wobei
die Klammerstücke (121, 121) angeordnet sind, um einander zugewandt zu sein, und wobei
der Vibrationskörper (13) an mindestens einem der Klammerstücke (121, 121) vorgesehen ist.

3. Hilfsmittel zur Linderung eines Nadeleinstichschmerzes (11) nach Anspruch 1 oder 2, wobei
der Vibrationskörper (13) an einer Oberflächenseite des Klammerstücks (121) eingerichtet ist, sodass er in der Lage ist, einen seitlichen Abschnitt eines Fingers zu berühren.

4. Hilfsmittel zur Linderung eines Nadeleinstichschmerzes (11) nach einem der Ansprüche 1 bis 3, wobei
der Vibrationskörper (13) an jedem der Klammerstücke (121, 121) vorgesehen ist.

5. Hilfsmittel zur Linderung eines Nadeleinstichschmerzes (11) nach einem der Ansprüche 1 bis 4, wobei
ein Abschnitt des Klammerstücks (121), das in der Lage ist, einen Finger zu berühren, in einer vorderen Oberfläche von diesem eine gewölbte konkave Oberfläche hat, die einer Form eines Fingers entspricht.

6. Hilfsmittel zur Linderung eines Nadeleinstichschmerzes (11) nach einem der Ansprüche 1 bis 5, wobei
ein Abschnitt des Klammerstücks (121), das in der Lage ist, einen seitlichen Abschnitt eines Fingers zu berühren, mit Konkavitäten und Konvexitäten an einer vorderen Oberfläche ausgebildet ist.

7. Einstichvorrichtung (100) mit einem Einstichhilfsmittel (1) mit einem Einstichabschnitt (124) und einer Einstichnadel (23) und dem Hilfsmittel zur Linderung eines Nadeleinstichschmerzes (11) nach einem der vorhergehenden Ansprüche, wobei
der Berührungsabschnitt (120) des Hilfsmittels zur Linderung eines Nadeleinstichschmerzes (11) ein Paar Klammerstücke (121, 121) hat, die mittels des Einstichabschnitts (124) entgegengesetzt angeordnet sind.

## Revendications

1. Outil (11) pour soulager une douleur de ponction d'aiguille à utiliser avec un outil de ponction pour ponctionner une surface d'une pulpe du doigt avec une aiguille de ponction pour soulager une douleur se produisant lorsque la surface de la pulpe du doigt est ponctionnée avec l'aiguille de ponction, ledit outil comprenant
une partie de contact (126) ayant un corps vibrant (13) et une paire d'éléments d'attache (121, 121) étant aptes à venir en contact avec des côtés opposés d'un doigt,
un vibreur (130) entraînant le corps vibrant (13), et une section de commande (15) adaptée pour commander un fonctionnement du vibreur (130) et pour faire varier un niveau des stimulations vibratoires du corps vibrant (13) avec le temps.

2. Outil (11) pour soulager une douleur de ponction d'aiguille selon la revendication 1, dans lequel
les éléments d'attache (121, 121) sont disposés pour se faire face, et dans lequel
le corps vibrant (13) est pourvu sur au moins l'un des éléments d'attache (121, 121).

3. Outil (11) pour soulager une douleur de ponction d'aiguille selon la revendication 1 ou 2, dans lequel
le corps vibrant (13) est disposé sur un côté de surface de l'élément d'attache (121) de manière à pouvoir venir en contact avec une partie latérale d'un doigt.

4. Outil (11) pour soulager une douleur de ponction d'aiguille selon l'une quelconque des revendications 1 à 3, dans lequel
le corps vibrant (13) est pourvu sur chacun des éléments d'attache (121, 121).

5. Outil (11) pour soulager une douleur de ponction d'aiguille selon l'une quelconque des revendications 1 à 4, dans lequel
une surface concave courbée correspondant à une forme de doigt se trouve sur surface avant d'une partie de l'élément d'attache (121) étant apte à venir en contact avec un doigt.

6. Outil (11) pour soulager une douleur de ponction d'aiguille selon l'une quelconque des revendications 1 à 5, dans lequel
une partie de l'élément d'attache (121) étant apte à venir en contact avec une partie latérale d'un doigt est formée avec des concavités et des convexités sur une surface avant.

7. Dispositif de ponction (100) comportant un outil de ponction (1) avec une partie de ponction (124) et une aiguille de ponction (23), et l'outil (11) pour soulager une douleur de ponction d'aiguille selon l'une quelconque des revendications précédentes, dans lequel
la partie de contact (120) de l'outil (11) pour soulager une douleur de ponction d'aiguille contient une paire d'éléments d'attache (121, 121) disposés de façon opposée à travers la partie de ponction (124).
